# EUROPÄISCHE PATENTSCHRIFT

(11) **EP 2 041 170 B1**
(45) Veröffentlichungstag und Bekanntmachung des Hinweises auf die Patenterteilung: **01.05.2013**
(21) Anmeldenummer: 07785903.1
(22) Anmeldetag: 05.07.2007
(51) Int. Cl.: C07K 14/62, C12P 21/02

(54) **VERFAHREN ZUR HERSTELLUNG VON INSULINANALOGA MIT DIBASISCHEM B-KETTENENDE**
METHOD FOR PRODUCING INSULIN ANALOGS HAVING A DIBASIC B CHAIN TERMINUS
PROCÉDÉ POUR PRODUIRE DES ANALOGUES D'INSULINE À EXTRÉMITÉ DE CHAÎNE B DIBASIQUE

(30) Priorität: 11.07.2006 DE 102006031955
(43) Veröffentlichungstag der Anmeldung: 01.04.2009
(73) Patentinhaber: Sanofi-Aventis Deutschland GmbH, 65929 Frankfurt am Main (DE)
(72) Erfinder: HABERMANN, Paul, 65926 Frankfurt am Main (DE); ZOCHER, Frank, 65926 Frankfurt am Main (DE)
(86) Internationale Anmeldenummer: PCT/EP2007/005933
(87) Internationale Veröffentlichungsnummer: WO 2008/006497

(56) Entgegenhaltungen:
- EP-A- 0 294 851
- EP-A1- 0 140 084
- EP-A2- 0 214 826
- EP-A2- 0 254 516
- WO-A-03/044210
- US-A- 5 656 722
- US-B1- 6 875 589
- LEYER S ET AL: "THE ROLE OF THE C-TERMINUS OF THE INSULIN B-CHAIN IN MODULATING STRUCTURAL AND FUNCTIONAL PROPERTIES OF THE HORMONE" INTERNATIONAL JOURNAL OF PEPTIDE AND PROTEIN RESEARCH, MUNKSGAARD, COPENHAGEN, DK, Bd. 46, Nr. 5, 1. November 1995 (1995-11-01), Seiten 397-407, XP000530836 ISSN: 0367-8377
- SCHELLENBERGER V ET AL: "PROTEASE-CATALYZED KINETICALLY CONTROLLED PEPTIDE SYNTHESIS" ANGEWANDTE CHEMIE. INTERNATIONAL EDITION, WILEY VCH VERLAG, WEINHEIM, DE, Bd. 30, Nr. 11, 1. November 1991 (1991-11-01), Seiten 1437-1449, XP000248151 ISSN: 1433-7851
- SCHELLENBERGER V ET AL: "ATTEMPTS FOR QUANTIFYING THE S' SUBSITE SPECIFICITY OF SERINE PROTEASES" ADVANCED IN THE BIOSCIENCES, PERGAMON PRESS, GB, Bd. 65, 1987, Seiten 159-166, XP009079584 ISSN: 0065-3446

### SEQUENCE LISTING

<110> Sanofi-Aventis Deutschland GmbH
<120> Verfahren zur Herstellung von Insulinanaloga mit dibasischem B-Kettenende
<130> DE2006/025
<140> 10 2006 031 955.9
   <141> 2006-07-11
<160> 20
<170> PatentIn version 3.3
<210> 1
   <211> 97
   <212> PRT
   <213> Artificial
<220>
   <223> Insulin-Fusionsprotein
<400> 1
   Met Ala Thr Thr Ser Thr Gly Asn Ser Ala Arg Phe Val Asn Gln His
   1               5                   10                  15
   Leu Cys Gly Ser His Leu Val Glu Ala Leu Tyr Leu Val Cys Gly Glu
               20                  25                  30
   Arg Gly Phe Phe Tyr Thr Pro Lys Thr Arg Arg Glu Ala Glu Asp Pro
           35                  40                  45
   Gln Val Gly Gln Val Glu Leu Gly Gly Gly Pro Gly Ala Gly Ser Leu
       50                  55                  60
   Gln Pro Leu Ala Leu Glu Gly Ser Leu Gln Lys Arg Gly Ile Val Glu
   65                  70                  75                  80
   Gln Cys Cys Thr Ser Ile Cys Ser Leu Tyr Gln Leu Glu Asn Tyr Cys
                   85                  90                  95
   Gly
<210> 2
   <211> 45
   <212> DNA
   <213> Artificial
<220>
   <223> Primer insu_a21_gly_rev
<400> 2
   ttttttaagc ttgtcgactc attagccgca gtagttctcc agctg 45
<210> 3
   <211> 45
   <212> DNA
   <213> Artificial
<220>
   <223> Primer ins_gly_a21_rev
<400> 3
   ttttttccat gggtcgacta tcagccacag tagttttcca gctgg 45
<210> 4
   <211> 48
   <212> DNA
   <213> Artificial
<220>
   <223> Primer alpha_insf1
<400> 4
   ttttttggat cctttggaat aaaagatttg ttaaccaaca cttgtgtg 48
<210> 5
   <211> 44
   <212> DNA
   <213> Artificial
<220>
   <223> Primer ins_gly_rev2
<400> 5
   ttttttccat gggtcgctat cagccacagt agttttccag ctgg 44
<210> 6
   <211> 49
   <212> DNA
   <213> Artificial
<220>
   <223> Primer ins_gly_rev2
<400> 6
   tttttggcgc cgaattcact actattagcc acagtagttt tccagctgg 49
<210> 7
   <211> 40
   <212> DNA
   <213> Artificial
<220>
   <223> Primer pich_insgly_dirf
<400> 7
   ttttttctcg agaaaagatt tgttaaccaa cacttgtgtg 40
<210> 8
   <211> 33
   <212> DNA
   <213> Artificial
<220>
   <223> Primer pich_insgly_dirrev
<400> 8
   ttttttggcg ccgaattcac tactattagc cac 33
<210> 9
   <211> 39
   <212> DNA
   <213> Artificial
<220>
   <223> Primer alpha_LT_H6_hirf1
<400> 9
   gcaccatcat caccatcact atactgactg cactgaatc 39
<210> 10
   <211> 48
   <212> DNA
   <213> Artificial
<220>
   <223> Primer alpha_LT_H6_hirf2
<400> 10
   gaaggggtac ctttggataa aagacttacg caccatcatc accatcac 48
<210> 11
   <211> 44
   <212> DNA
   <213> Artificial
<220>
   <223> Primer Hir_entero_insf
<400> 11
   cttcaggacg atgacgataa atttgttaac caacacttgt gtgg 44
<210> 12
   <211> 41
   <212> DNA
   <213> Artificial
<220>
   <223> Primer Hir_entero_insrev
<400> 12
   tttatcgtca tcgtcctgaa ggctgaaggt attcctcagg g 41
<210> 13
   <211> 23
   <212> DNA
   <213> Artificial
<220>
   <223> Primer alpha_lt_enterof
<400> 13
   gaaggggtac ctttggataa aag 23
<210> 14
   <211> 5
   <212> PRT
   <213> Artificial
<220>
   <223> Erkennungssequenz
<400> 14
   Asp Asp Asp Asp Lys
1 5
<210> 15
   <211> 44
   <212> DNA
   <213> Artificial
<220>
   <223> Primer Desphef1
<400> 15
   cttcagggaa attcggcacg agttaaccaa cacttgtgtg gttc 44
<210> 16
   <211> 44
   <212> DNA
   <213> Artificial
<220>
   <223> Primer Desphe_rev1
<400> 16
   gaaccacaca agtgttggtt aactcgtgcc gaatttccct gaag 44
<210> 17
   <211> 45
   <212> DNA
   <213> Artificial
<220>
   <223> Primer Ala_b31f1
<400> 17
   cttctacact ccaaagacgg ctcgtggtat cgttgaacaa tgttg 45
<210> 18
   <211> 45
   <212> DNA
   <213> Artificial
<220>
   <223> Primer Ala_b31rev1
<400> 18
   caacattgtt caacgatacc acgagccgtc tttggagtgt agaag 45
<210> 19
   <211> 42
   <212> DNA
   <213> Artificial
<220>
   <223> Primer Lys_b31f
<400> 19
   cttctacact ccaaagacga aaggtatcgt tgaacaatgt tg 42
<210> 20
   <211> 42
   <212> DNA
   <213> Artificial
<220>
   <223> Primer Lys_b31rev
<400> 20
   caacattgtt caacgatacc tttcgtcttt ggagtgtaga ag 42

## Beschreibung

Die Erfindung betrifft ein Verfahren zur Herstellung eines Insulins mit dibasischem Kettenende, indem man eine Vorstufe davon biotechnologisch herstellt und anschließend in einer enzymkatalysierten Ligationsreaktion mit Lysinamid oder Argininamid, oder durch Schutzgruppen modifiziertes Lysin oder Arginin, und optional anschließender Hydrolyse, zu diesem Insulin umsetzt.

Weltweit leiden etwa 177 Mio. Menschen an Diabetes mellitus. Darunter sind etwa 17 Mio. Typ I-Diabetiker, für die die Substitution der fehlenden endokrinen Insulinsekretion die einzige derzeit mögliche Therapie darstellt. Die Betroffenen sind lebenslang, in der Regel mehrmals täglich, auf Insulininjektionen angewiesen. Im Gegensatz zum Typ I-Diabetes besteht beim Typ II-Diabetes nicht grundsätzlich ein Mangel an Insulin, jedoch wird in einer Vielzahl von Fällen, vor allem im fortgeschrittenen Stadium, die Behandlung mit Insulin, gegebenenfalls in Kombination mit einem oralen Antidiabetikum, als günstigste Therapieform angesehen.

Beim Gesunden ist die Insulinfreisetzung strikt an die Konzentration der Blutglucose gekoppelt. Erhöhte Blutglucosespiegel, wie sie nach Mahlzeiten auftreten, werden durch eine entsprechende Steigerung der Insulinsekretion rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glucose zu gewährleisten und die hepatische Glucoseproduktion in der Nacht niedrig zu halten. Der Ersatz der körpereigenen Insulinsekretion durch exogene, meist subkutane Applikation von Insulin erreicht in der Regel die oben beschriebene Qualität der physiologischen Regulation der Blutglucose nicht annähernd. Häufig kommt es zu Entgleisungen des Blutglucosespiegels nach oben oder unten, die in ihren schwersten Formen lebensbedrohlich sein können. Daneben stellen jedoch auch über Jahre erhöhte Blutglucosespiegel ohne anfängliche Symptome ein erhebliches Gesundheitsrisiko dar. Die großangelegte DCCT-Studie in den USA (The Diabetes Control and Complications Trial Research Group (1993) N. Engl. J. Med. 329, 977-986) wies eindeutig nach, dass chronisch erhöhte Blutglucosespiegel wesentlich für die Entwicklung diabetischer Spätschäden verantwortlich sind. Diabetische Spätschäden sind mikro- und makrovaskuläre Schädigungen, die sich u. U. als Retino-, Nephro-, oder Neuropathie manifestieren und zu Erblindung, Nierenversagen sowie dem Verlust von Extremitäten führen und darüber hinaus mit einem erhöhten Risiko für Herz/Kreislauferkrankungen einhergehen. Daraus ist abzuleiten, dass eine verbesserte Therapie des Diabetes in erster Linie darauf abzielen muss, die Blutglucose möglichst eng im physiologischen Bereich zu halten. Nach dem Konzept der intensivierten Insulintherapie soll dies durch mehrmals tägliche Injektionen von schnell und langsam wirkenden Insulinzubereitungen erreicht werden. Rasch wirkende Formulierungen werden zu den Mahlzeiten gegeben, um den postprandialen Anstieg der Blutglucose auszugleichen. Langsam wirkende Basalinsuline sollen die Grundversorgung mit Insulin insbesondere während der Nacht sicherstellen, ohne zu einer Hypoglykämie zu führen.

Insulin ist ein Polypeptid aus 51 Aminosäuren, die sich auf 2 Aminosäureketten verteilen: die A Kette mit 21 Aminosäuren und die B-Kette mit 30 Aminosäuren. Die Ketten sind durch 2 Disulfidbrücken miteinander verbunden. Insulinzubereitungen werden seit vielen Jahren zur Diabetestherapie eingesetzt. Dabei werden nicht nur natürlich vorkommende Insuline verwendet, sondern neuerdings auch Insulinderivate und -analoga.

Insulinanaloga sind Analoga von natürlich vorkommenden Insulinen, nämlich Humaninsulin oder tierischen Insulinen, welche sich durch Substitution wenigstens eines natürlich auftretenden Aminosäurerestes mit anderen Aminosäureresten und/oder Addition/Entfernen wenigstens eines Aminosäurerestes von dem entsprechenden, ansonsten gleichen natürlich vorkommenden Insulin unterscheiden. US 5,656,722 z. B. beschreibt des Phe ^{(B1)} - Insulinderivate. Es kann sich bei den hinzugefügten und / oder ersetzten Aminosäureresten auch um solche handeln, die nicht natürlich vorkommen.

Insulinderivate sind Derivate von natürlich vorkommenden Insulinen oder Insulinanaloga, bei denen einer oder mehrere Aminosäurereste und/oder die N- oder C-Termini der A- und/oder B-Kette durch funktionelle Gruppen substituiert sind. Die funktionellen Gruppen sind ausgewählt aus einer Gruppe enthaltend Amidreste, Aminreste, Carboxlyreste, Alkylreste, Alkoholreste und Alkoxyreste.

Eine effektive Insulintherapie macht Gebrauch von sogenannten Basal - Insulinen. Darunter versteht man Formulierungen, die ein langsames, kontinuierliches Freisetzen von exogen appliziertem Insulin ermöglichen. Dadurch wird über längere Zeit eine basale Insulinkonzentration im Körper erreicht, die sich vorteilhaft auf den physiologischen Zustand des an Diabetes erkrankten Menschen auswirkt.

Das rekombinante Insulin Analog Gly (A21), Arg(B31), Arg (B32) Humaninsulin (Insulin Glargin) zeichnet sich dabei dadurch aus, dass es nur alle 24 Stunden - also nur einmal am Tag - dem Körper zugeführt werden muss, um eine basale Wirkung zu erreichen. Die Einmal-pro-Tag Applikation führt zu einer verbesserten Lebensqualität. Die verbesserte Physiologie führt z.B. zu einer Verringerung des Hba1c Wertes und man kann erwarten, dass sich aufgrund dieser Verbesserung diabetische Spätfolgen - wenn überhaupt - erst wesentlich später einstellen, wodurch sich die Lebenserwartung der betroffenen Diabetiker verlängern kann.

Entsprechend groß ist der Bedarf an diesem Insulinanalogon. Da die Zahl der Diabetiker ständig steigt, ist es zudem von wirtschaftlichem Interesse, die Herstellkosten für entsprechende Analoga zu minimieren. In US 5,656,722 ist die mögliche Herstellung von Insulinanaloga über ein Präproinsulinfusionsprotein beschrieben, das aus einem Fusionsteil ("Präteil") und einer Affenproinsulinvariante besteht. Eines der beschriebenen Analoga enthält in Position A(21) Glycin statt Asparagin. Das entsprechende Fusionsprotein ist eine Peptidvorläufervariante für die Herstellung von Insulin Glargin. Das Verfahren sieht vor, dass man aus diesem Fusionsprotein den Präteil und das C-Peptid durch Umsetzung mit Trypsin entfernt. EP-A 0 668 292 beschreibt ein Fusionsprotein das dem gleichen Prinzip folgt, aber ein gegenüber US 5,656,722 verbessertes Verfahren zur Herstellung von Insulin Glargin erlaubt. Dabei ist es dem Fachmann klar, dass besonders an der Grenze von der Insulin B- und der C-Kette, die durch die dibasische Struktur Arg-Arg definiert ist , eine partielle Spaltung möglich ist, die zu einem B31 mono -Arg Humaninsulinanalog führt. Dieses Fehlprodukt muss von der eigentlichen Verbindung von Interesse abgetrennt werden. Dies führt zu einer deutlichen Beeinträchtigung der Ausbeute. Man kann das Problem so umgehen, dass man gentechnisch Proinsuline herstellt und diese mit einer spezifischen Endoprotease, wie z. B. Lysyl- Endopeptidase umsetzt und das entstandene des-B30 Humaninsulin(analog) in einem peptidchemischen Semisyntheseansatz mit dem Tripeptid Thr-Arg-Arg umsetzt. EP-A 0 132 769 und WO 2003/044210 beschreiben, dass die reaktiven Gruppen des Tripeptides bei der Reaktion geschützt vorliegen müssen. Anschließend an die Reaktion werden die Schutzgruppen abgespalten. Dieser Weg ist mit Kosten verbunden, die durch die Darstellung des Tripeptides über chemische Synthese und das Einführen von Schutzgruppen entstehen. Es wäre also ein Verfahren wünschenswert, dass die Herstellung von Arg(B31), Arg (B32) -Insulinanaloga aus der Arg(B31) Humaninsulin - Vorstufe erlaubt.

Die deutsche Patentanmeldung Nr. 10 2005 046 113.1 (nicht veröffentlicht) beschreibt ein Verfahren, dass die Trypsin-katalysierte Ligation von Aminosäuren, die C-terminal amidiert sind, an Peptide, deren C-terminale Aminosäure aus Lysin oder Arginin besteht, beinhaltet. Die Ausbeuten, die dabei beobachtet werden sind überraschend hoch und zudem kann die Kupplungsreaktion ohne die Maskierung mit Schutzgruppen vorgenommen werden. Die Reaktion findet in nichtwässrigem Milieu statt. Es wurde nun überraschend gefunden, dass die Kupplung von Arginin - Amid oder Lysin Amid an B31-Insulinanaloge mit hohen Ausbeuten möglich ist. Dabei kann man die Reaktion überraschend so steuern, dass bevorzugt Insulinanaloga der Form Arg(B31), Arg (B32) - Humaninsulinamid bzw. der Form Arg(B31), Lys (B32) - Humaninsulinamid entstehen. Die Ausbeute ist dabei größer als 60%. Am Ende der Reaktion kann die Amidgruppe durch saure Hydrolyse abgespalten. Es wurde ebenfalls überraschend gefunden, dass man alternativ zu Lysin - oder Argininamid bei der Reaktion Arginin oder Lysin einsetzen kann, das eine Schutzgruppe tragen kann. Als Beispiel für Schutzgruppen seien t-Butyloxycarbonyl (Boc) oder Dimethoyphenylpropyloxycarbonyl (DZZ) benannt. Da in der Literatur beschrieben wird, dass insbesondere geschützte Argininderivate in verschiedenen Lösungsmitteln instabil sein können, ist es dem Fachmann klar, dass in der Peptidchemie stetig neue Schutzgruppen entwickelt werden, die eine verbesserte Stabilität bewirken. Entsprechend der Schutzgruppen oder Amidgruppe kann durch Variation der Reaktionsbedingungen die Ausbeute positiv beeinflusst werden. Dies ist dem Fachmann geläufig und auch Gegenstand der Erfindung. Damit wird das partielle Spaltprodukt B(31) Humaninsulin, das bei Herstellung von Insulin Glargin oder vergleichbaren Arg(B31), Arg (B32) - Insulinanaloga aus Präproinsulinvorstufen (US 5,656,722) für die Herstellung des Wertstoffes zugänglich. Entsprechende Fusionsproteine müssen dabei nicht intrazellulär hergestellt werden. Es ist dem Fachmann klar, dass Proinsulinanaloga auch durch bakterielle Expression mit anschließender Sekretion in das Periplasma und/oder in den Kulturüberstand hergestellt werden können. Die Europäische Patentanmeldung EP-A 1 364 029 beschreibt dies beispielhaft. Auch die Verwendung von Arg(B31) - Humaninsulin - Vorstufen, die direkt nach Expression aus solchen bakteriellen Verfahren entstehen, ist Gegenstand der Erfindung.

Es ergibt sich zusätzlich ein weiterer Verfahrenstechnischer Aspekt, der ebenfalls Gegenstand der Erfindung ist. Die Europäische Patentanmeldung EP-A 0 347 781, bzw. die Europäischen Patentanmeldungen EP-A 1 364 030 und EP-A 1 364 032 beschreiben Hefe-basierte Verfahren zur Herstellung von Miniproinsulinen mit hohen Ausbeuten. Wenn man ein solches oder ähnliches Verfahren auf die Herstellung von Miniproinsulinen, die in Position A21 die im US-Patent 5,656,722 beschriebenen Aminosäurereste, also Gly, Ala, Val, Leu, Ile, Pro, Phe, Trp, Met, Ser, Thr, Cys, Tyr, Asp oder Glu, tragen, erweitert, dann lassen sich diese Miniproinsuline direkt nach Spaltung in das zweikettige Insulin in die Arg(B31), Arg (B32) -Insulinanaloga umwandeln.

Erfolgt die Expression, wie in EP-A 1 364 032 beschrieben, über ein Fusionsprotein, dann ist es vorteilhaft, den Präteil nicht über Trypsin oder ähnliche Endoproteasen abzuspalten. Statt dessen wird eine Spaltstelle eingebaut, die durch eine spezifische Endoprotease, die nicht das Insulinderivat spaltet, erkannt wird, um entsprechend den Prä - bzw. Fusionsteil abzuspalten. Beispielhaft werden Enterokinase (DDDDK) oder FaktorXa (IEGR) benannt. Auch dies ist Gegenstand der Erfindung. Dabei ist dem Fachmann klar, dass die beiden Spaltreaktionen in einer Eintopfreaktion ablaufen können. Eine weitere Möglichkeit ergibt sich, indem man den Fusionsteil erst in einem Folgeschritt abspaltet. Als Fusionsproteinteil kann man dabei Derivate von einer Vielzahl von gut sekretierten Proteinen wählen. Für Bakterien seien beispielhaft DHFR (Dihydrofolat Reduktase), Glutathione S- Transferase und Hirudin genannt. Für Hefesekretion kann man z.B. Albumin oder Derivate hiervon, Superoxid Dismutase oder Derivate, Interleukin 2 oder Derivate und Hirudin oder Derivate verwenden. In der vorliegenden Anmeldung wird beispielhaft ein Hirudinderivat als Fusionsteil sowohl zur bakteriellen Expression als auch zur Hefeexpression verwendet. Dabei wurde überraschend gefunden, dass man die Hirudinsequenz weiter verändern kann, indem man eine Peptidsequenz aus Histidinen in Folge und / oder eine Peptidsequenz DDDDK, die die Erkennungsstelle für Enterokinase darstellt, einführt, ohne das Faltung des Miniproinsulinteils beeinträchtigt wird. Damit werden Methoden der Affinitätschromatographie zugänglich. Auch dies ist Gegenstand der Erfindung.

Dem Fachmann ist ferner geläufig, dass die beispielhaft beschriebenen Expressionssysteme nur einen kleinen Ausschnitt der für die rekombinante Herstellung von Proteinen entwickelten Wirts/Vektorsysteme darstellen. WirtsNektor Systeme, die die Herstellung der Zielpeptide erlauben, sind somit auch Bestandteil der Erfindung.

Gegenstand der Erfindung ist also die Herstellung von Insulinanaloga, die durch das Vorhandensein der Aminosäurereste Arg(B31), Arg (B32) oder Arg(B31), Lys (B32) charakterisiert sind, aus Arg(B31) - Humaninsulin - Vorläufern der Analoga über Trypsin - katalysierte Ligation mit Arginin oder Lysin. Dabei ist dem Fachmann klar, dass aufgrund der überraschenden Selektivität der Reaktion die Ligationsreaktion auch über mehrere Reaktionszyklen wiederholt werden kann, so dass Insulinanaloga zugänglich werden, die über die Positionen B31 und B32 hinaus weitere basische Aminosäuren Lysin oder Arginin tragen. Dies erreicht man, indem man eine Kupplungsreaktion durchführt, die endständige Aminosäure deamidiert oder entschützt und das Produkt erneut in einen entsprechenden Folgereaktionszyklus einsetzt. Zu solchen Produkten kann man ebenfalls gelangen, wenn man ein Analogon, das bereits Arg(B31), Arg (B32) bzw. Arg(B31), Lys(B32) trägt, als Vorstufe benutzt. Ebenfall darstellen lassen sich Analoga, die in Position B31 und folgend beliebige genetisch kodierbare Aminosäuren enthalten die, nicht Arginin oder Lysin in Folge sein müssen, deren C-terminales Ende aber durch die di-basische Sequenz Arg-Arg, Arg-Lys, Lys-Lys oder Lys-Arg gekennnzeichnet ist.
Dabei ist die Reaktion im Hinblick auf die Verwendung von Trypsin als Katalysator nicht begrenzt. Dem Fachmann ist geläufig, dass man neben den bekannten kommerziell verfügbaren Trypsinen aus Ratte, Rind, Schwein, Mensch oder anderer Isoenzyme oder deren Derivaten oder Varianten auch folgende Enzyme verwenden kann: Cathepsin, Trypsin aus Fusarium oxysporum und aus Streptomyces (S. griseus, S. exfoliatus, S. erythraeus, S. fradiae und S. albidoflavus), Tryptase, Mastin, Acrosin, Kallikrein, Hepsin, Prostasin I, Lysyl endopeptidase (Lysin -C) und Endoproteinase-Arg-C (Clostripain).

Gegenstand der Erfindung ist daher ein Verfahren zur Herstellung eines Insulinanalogons oder eines Derivates davon, bei dem
an ein Ausgangs-Insulinanalogon oder ein Derivat davon, dessen C-terminale Aminosäure der A- und/oder B-Kette ausgewählt ist aus einer Gruppe enthaltend natürlich vorkommende, basische Aminosäuren oder deren Analoga oder Derivate, an eine der genannten C-terminalen Aminosäuren
in Gegenwart eines Enzyms mit der biologischen Aktivität von Trypsin
eine amidierte oder C-terminal mit einer Schutzgruppe geschützte, natürlich vorkommende, basische Aminosäure ausgewählt aus der Gruppe enthaltend Lys und Arg addiert,
und das erhaltene modifizierte Insulinanalogon aufreinigt und optional die Amidgruppe oder C-terminale Schutzgruppe der addierten Aminosäure oder des addierten Peptids abspaltet,
wobei das Insulinanalogon charakterisiert ist durch die allgemeine Formel I 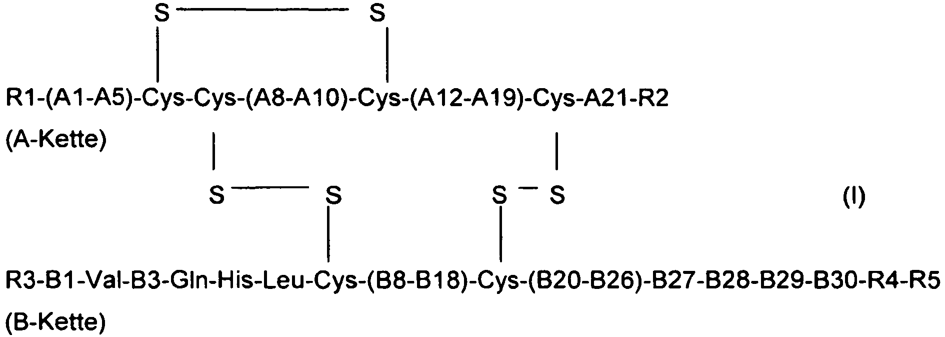 worin bedeuten

| | |
|---|---|
| (A1-A5) | die Aminosäurereste in den Positionen A1 bis A5 der A-Kette von Humaninsulin oder tierischem Insulin, |
| | |
| (A12-A19) | die Aminosäurereste in den Positionen A12 bis A19 der A-Kette von Humaninsulin oder tierischem Insulin, |
| | |
| A21 | ein natürlich auftretender Aminosäurerest, |
| | |
| (B8-B18) | die Aminosäurereste in den Positionen B8 bis B18 der B-Kette von Humaninsulin oder tierischem Insulin, |
| (B20-B26) | die Aminosäurereste in den Positionen B20 bis B26 der B-Kette von Humaninsulin oder tierischem Insulin, |
| (A8-A10) | die Aminosäurereste in den Positionen A8 bis A10 der A-Kette von Humaninsulin oder tierischem Insulin, |
| | |
| B30 | eine chemische Bindung oder ein natürlich auftretender Aminosäurerest, |
| | |
| B1 | eine chemische Bindung oder ein natürlich auftretender Aminosäurerest, |
| | |
| B3 | ein natürlich auftretender Aminosäurerest, |
| | |
| B27, B28 und B29 | ein natürlich auftretender Aminosäurerest, |
| | |
| R1 | eine Aminogruppe oder ein bis drei natürlich auftretende Aminosäurereste, |
| | |
| R2 | eine Carboxygruppe oder ein bis drei natürlich auftretende Aminosäurereste, |
| | |
| R3 | eine Aminogruppe oder ein bis drei natürlich auftretende Aminosäurereste, |
| | |
| R4 | eine basische Aminosäure darstellt, |
| | |
| R5 | einen basischen Aminosäurerest, dessen C-Terminus entweder frei oder amidiert vorliegt, |

wobei derjenige Aminosäurerest, an dessen C-Terminus der N-Terminus von R5 anschließt, ausgewählt ist aus einer Gruppe enthaltend Arg und Lys; und
wobei das Ausgangs-Insulinanalogon charakterisiert ist durch die allgemeine Formel II 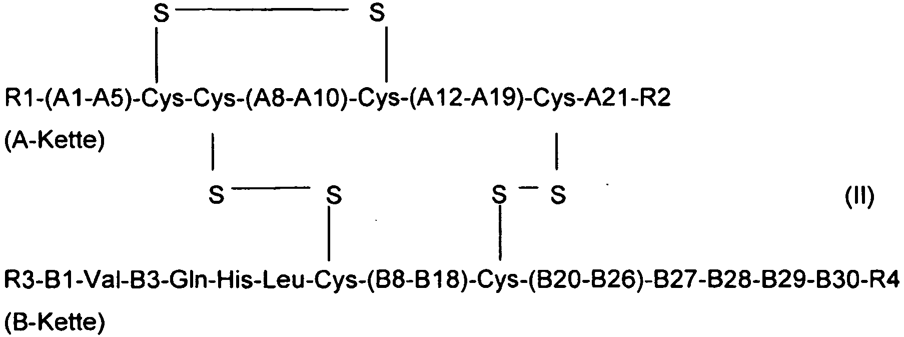 wobei R1, (A1-A5), (A8-A10), (A12-A19), A21, R2, R3, B1, B3, (B8-B18), (B20-B26), B27, B28, B29, B30 und R4 definiert sind wie in Anspruch 1 und der C-terminale Aminosäurerest der B-Kette ausgewählt ist aus einer Gruppe enthaltend Arg und Lys.

Ein weiterer Gegenstand der Erfindung ist ein Verfahren wie oben beschrieben, wobei die amidierte oder C-terminal mit einer Schutzgruppe geschützte, natürlich vorkommende, basische Aminosäure C-terminal mit einer Boc-Schutzgruppe C-terminal geschütztes Arginin ist.

Ein weiterer Gegenstand der Erfindung ist ein Verfahren wie oben beschrieben, wobei das modifizierte Insulinanalogon Gly(A21), Arg(B31), Arg(B32) Humaninsulin ist, dessen C-terminales Ende der B-Kette amidiert ist, bei dem insbesondere das Ausgangs-Insulinanalogon Gly(A21), Arg(B31) Humaninsulin ist.

Ein weiterer Gegenstand der Erfindung ist ein Verfahren wie oben beschrieben, wobei das Ausgangs-Insulinanalogon hergestellt wird durch rekombinante Expression eines Vorläuferproteins enthaltend die A- und die B-Kette des Ausgangs-Insulinanalogons, insbesondere ein solches Verfahren, wobei ein Gen, welches Teil eines Replicons ist, exprimiert wird.

Ein weiterer Gegenstand der Erfindung ist ein Verfahren wie oben beschrieben, wobei als Wirtszelle ein Bakterium oder eine Hefe verwendet wird.

Ein weiterer Gegenstand der Erfindung ist ein Verfahren wie oben beschrieben, wobei das Vorläuferprotein nach der Expression sekretiert wird, insbesondere wobei das Vorläuferprotein aus dem zellulären Überstand von Bakterien oder Hefen isoliert wird.

Ein weiterer Gegenstand der Erfindung ist ein Verfahren wie oben beschrieben, wobei das Vorläuferprotein aus dem Periplasma eines Bakteriums isoliert wird.

Ein weiterer Gegenstand der Erfindung ist ein Verfahren wie oben beschrieben, wobei das nach einem der genannten Ansprüche erhaltene Vorläuferprotein einem Faltungsprozeß und enzymatischer Spaltung unterworfen wird.

Ein weiterer Gegenstand der Erfindung ist ein Verfahren wie oben beschrieben, wobei das Ausgangs-Insulinanalogon hergestellt wird durch rekombinante Direktexpression.

Ein weiterer Gegenstand der Erfindung ist ein Verfahren wie oben beschrieben, wobei das Enzyms mit der biologischen Aktivität von Trypsin ausgewählt ist aus einer Gruppe enthaltend menschliches Trypsin, Schweinetrypsin, Rindertrypsin und eine Variante von menschlichem Trypsin, Schweinetrypsin und Rindertrypsin.

Ein weiterer Gegenstand der Erfindung ist ein Verfahren wie oben beschrieben, wobei das C-terminale Ende der B-Kette des modifizierten Insulinanalogons nachfolgend in einer Hydrolysereaktion entschützt wird.

Ein weiterer Gegenstand der Erfindung ist ein Verfahren wie oben beschrieben, bei dem das erhaltene Insulinanalogon Gly(A21), Arg(B31), Arg(B32) Humaninsulin ist.

Die Erfindung wird im folgenden anhand einiger Durchführungsbeispiele näher erläutert. Diese Durchführungsbeispiele sollen nicht beschränkend wirken.

### Beispiel 1: Herstellung von Arg(B31), Gly(A21) Insulin aus einem Fusionsprotein nach in vitro Faltung

Die Patentschrift US 5,663,291 beschreibt in ihrem Beispiel 1 die Gewinnung eines korrekt gefalteten Insulin- Fusionsproteins der Struktur:

Dieses Material wird entsprechend Beispiel V der Patentschrift US 5,227,293 über Umsetzung mit Trypsin in zwei-kettiges Insulin umgesetzt und Arg(B31), Arg(B32), Gly(A21) Insulin und Arg(B31), Gly(A21) Insulin isoliert.

Somit kann man das Arg(B31), Arg(B32), Gly(A21) - Insulinanalog direkt erhalten werden, während das Arg(B31), Gly(A21) - Nebenprodukt als Vorstufe in die Trypsin katalysierte Ligation mit modifiziertem Arginin bzw. Lysin eingesetzt werden kann.

### Beispiel 2: Herstellung von Arg(B31), Gly(A21) Insulin aus einem durch Sekretion gewonnenen Fusionsproteins, das Proinsulin korrekt gefaltet enthält

Alternativ zu Beispiel 1 lassen sich Fusionsproteine auch durch Sekretion in bakteriellen Systemen herstellen. Dabei wird die Proinsulinstruktur als Teil des Fusionsproteins korrekt gefaltet und man kann auf den 'in vitro' Rückfaltungsschritt verzichten. Die Patentanmeldung WO 02/068660 schlägt ein solches System vor. Wenn man z.B. in dem in Beispiel 1 dieser internationalen Patentanmeldung beschriebenen Plasmid pBpfuHir_Ins das Codon für Asn (A21) durch ein Codon für Gly(A21) ersetzt gelangt man zu einem Fusionsprotein aus dem man beispielhaft Insulin Glargin gewinnen kann und dabei als Nebenprodukt, wie in Beispiel 1 beschrieben, Arg(B31), Gly(A21) Humaninsulin isolieren kann.

Zur Herstellung der Sequenz benötigt man einen neuen Primer insu_a21_gly_rev mit folgender Struktur:
5'- TTTTTTAAGCTTGTCGACTCATTAGCC GCAGTAGTTCTCCAGCTG-3' (SEQ ID NO.: 2)

Dieser Primer wird in Analogie zu der Patentanmeldung WO 02/068660 mit dem Primer pfu1 auf DNA des Plasmides pBpfuHir_ins in einer PCR eingesetzt. Aus dem PCR - Produkt lässt sich ein BamH1/Hind3 Fragment isolieren, dass man entsprechend dem Beispiel der Patentanmeldung WO 02/068660 klonieren kann. Nach Expression wird ein Fusionsprotein isoliert, das entsprechend Beispiel 1 der vorliegenden Anmeldung weiter behandelt wird.

Dem Fachmann ist klar, dass man auch die Vorstufe Arg(B31), Gly(A21) Humaninsulin direkt durch bakterielle Sekretion eines Fusionsproteins gewinnen kann. Auch dies ist Gegenstand der Erfindung.

### Beispiel 3: Herstellung von Arg(B31), Arg(B32), Gly(A21) -Insulin aus einer Arg(B31), Gly(A21) - Vorstufe über Kupplung mit Argininamid

100 mg 21A-Gly-30B L-Arg-Insulin werden in 0.95 ml Argininamidlösung (446 g/L) gelöst , 0.13 mL M Na-Acetatpuffer (pH 5.8) und 2 ml DMF zugegeben. Die Reaktionsmischung wird auf 12° C gekühlt und durch Zugabe von 0.094 ml Trypsin (0.075 mg, Roche Diagnostics) gestartet.

Nach 8 h wird die Reaktion durch Zugabe von TFA bis pH 2.5 gestoppt und per HPLC analysiert. Es bildet sich >60 % -Arg(B31), Arg(B32), Gly(A21) Humaninsulin. Nach Zusatz von Trypsininhibitorlösung erfolgt die Reinigung des amidierten Analogs in Analogie zu US 5,656,722. Das amidierte Insulinanalog wird anschließend in Gegenwart von Säure mehrere Stunden zu Arg(B31), Arg (B32), -Gly(A21) Humaninsulin hydrolysiert.

### Beispiel 4: Herstellung von Arg(B31), Lys(B32), Gly(A21) Humaninsulin aus einer Arg(B31), Gly(A21) Humaninsulin - Vorstufe über Kupplung mit Lysinamid

100 mg 21A-Gly-30B L-Arg-Insulin werden in 0.93 ml Lysinamidlösung (400 g/L) gelöst , 0.13 mL M Na-Acetatpuffer (pH 5.8) und 2 ml DMF zugegeben. Die Reaktionsmischung wird auf 12° C gekühlt und durch Zugabe von 0.094 ml Trypsin (0.075 mg, Roche Diagnostics) gestartet.

Nach 8 h wird die Reaktion durch Zugabe von TFA bis pH 2.5 gestoppt und per HPLC analysiert. Es bildet sich Arg(B31), Lys(B32)-NH₂, Gly (A21) Humaninsulin, das nach Zusatz von Trypsininhibitorlösung in Analogie zu US 5,656,722 gereinigt wird. Das amidierte Insulinanalog wird anschließend in Gegenwart von Säure mehrere Stunden zu Arg(B31), Lys(B32), Gly(A21) Humaninsulin hydrolysiert.

### Beispiel 5: Herstellung von Arg(B31), Arg(B32), Gly(A21)-Insulin aus einer Arg(B31), Gly(A21) - Vorstufe über Kupplung mit H- Arg (Boc)2-OH

0,25mg Arg(B31), Gly(A21) Humaninsulin werden in einem Eppendorfgefäß mit 11µl 0,1 M Pyridin-Acetatpuffer (pH5,6), 60µl einer 130g/L Lösung von H-Arg(Boc)2-OH x HCl in 0,1 M Pyridin - Acetatpuffer (pH5,6) und 119µl DMF versetzt und bei 12°C über einige Stunden mit Trypsin (Roche Diagnostics) inkubiert.

Die Reaktion wird durch Zusatz eines Gemisches aus 25% Wasser, 25% Acetonitrile und 50% Trifluoressigsäure gestoppt. Das Gemisch wird lyophilisiert und zur Abspaltung der Schutzgruppe in 1ml TFA gelöst und ca 3 Stunden bei Raumtemperatur stehen gelassen. Die Reinigung des Arg(B31), Arg(B32)- NH₂, Gly(A21) Humaninsulins erfolgt beispielhaft in Analogie zu US 5,656,722.

### Beispiel 6: Herstellung von Arg(B31), Lys(B32), Gly(A21)Insulin aus einer Arg(B31), Gly(A21) - Vorstufe über Kupplung mit H - Lys (Boc)-OtBu

50 mg Arg(B31), -Gly(A21) Humaninsulin werden in 0,62 ml H-Lys (Boc)-OtBu Lösung (0,5 g/mL, pH 5) gelöst und 1 ml N,N-Dimethylformamid (DMF) zugesetzt. Das Gemisch wird auf 12°C gekühlt und 2mg Trypsin (Roche Diagnostics) zugesetzt.

Nach mehr als 10 Stunden wird die Reaktion durch Zugabe von 2 ml eines 50%-igen Acetonitril /Wasser - Gemisches und 1 ml TFA (100%) abgestoppt. Das Gemisch wird lyophilisiert und zur Abspaltung der Boc-Schutzgruppe in 1ml TFA gelöst und ca. 3 Stunden bei Raumtemperatur stehen gelassen. Die Reinigung des Arg(B31), Lys(B32), OH erfolgt beispielhaft in Analogie zu US 5,656,722.

### Beispiel 7: Gensequenz zu Sekretion eines Hirudin Arg(B31), Gly(A21) Insulin - Fusionsproteins durch Bäckerhefe

Die Patentanmeldung EP-A 1 364 032 schlägt die Verwendung von Hirudin als Fusionspartner zur Expression und Sekretion von anderen pharmazeutisch interessanten Wertproteinen in Hefen vor.

Beispiel 1 der Patentanmeldung EP-A 1 364 032 beschreibt ein Wirtsvektorsystem zur Herstellung eines Fusionsproteins, das aus einem Hirudinderivat und Miniproinsulin besteht. Dieses System kann man beispielhaft für die Herstellung von Miniproinsulinen, die in Position A21 die Aminosäure Asparagin durch Aminosäuren, wie sie in US 5,656,722 beschrieben sind, verwenden.

Die Konstruktion des Expressionsvektors kann in Analogie zu dem Beispiel der Patentanmeldung EP-A 1 364 032 erfolgen, wenn man den Primer insnco1 rev ersetzt und so konzipiert, dass das Codon in Position A21 verändert wird.

Zur Herstellung der für Arg(B31), Gly A(21) Humaninsulin kodierenden Sequenz wird z.B. folgender Primer synthetisiert:
ins_gly_a21_rev
5'-TTTTTTCCATGGGTCGACTATCAGCCACAGTAGTTTTCCAGCTGG-3' (SEQ ID NO.: 3)

Der Primer überdeckt dabei vollständig den für die Aminosäuren A15-A21 des Insulinanalogs kodierenden Genabschnitt. Kombiniert man diesen Primer mit dem Primer SEQ ID NO:4 aus Beispiel 1 der Anmeldung und verwendet das Plasmid pADH2Hir_ins als 'Template', so lässt sich über PCR ein DNA Fragment generieren, dass nach Verdau mit den Restriktionsenzymen Kpnl und NcoI in den entsprechend geöffneten Expressionsvektor insertiert wird und das gewünschte Fusionsprotein enthält.

Der Vektor erhält die Bezeichnung pADH2Hir_ins_glyA21. Das Fusionsprotein wird entsprechend der der Patentanmeldung EP-A 1 364 032 exprimiert und prozessiert zu Gly(A21) - Miniproinsulin, das entsprechend Beispiel 2 in Arg(B31), Lys(B32), Gly(A21) Humaninsulin umgewandelt wird.

### Beispiel 8: Gensequenz zur direkten Sekretion der Arg(B31), Gly(A21) - Vorstufe durch Bäckerhefe

DNA des in Beispiel 7 beschriebenen Plasmides pADH2Hir_ins_glyA21 wird zur Herstellung eines Vektorkonstruktes zur direkten Sekretion von Arg(B31), Gly A(21) Humaninsulin verwendet.

Folgende Primer werden synthetisiert.
alpha_insf1
5'- TTTTTTGGATCCTTTGGAATAAAAGATTTGTTAACCAACACTTGTGTG-3'(SEQ ID NO.: 4)
   Er überdeckt die Sequenz des C-Terminus des alpha - Faktors, Codone für Lys-Arg und des N-Terminus der Miniproinsulin Sequenz.
ins_gly_rev2
5'- TTTTTTCCAT GGGTCGCTAT CAGCCACAGT AGTTTTCCAG CTGG -3' (SEQ ID NO.: 5)

Der Primer hybridisiert mit dem 3'Ende der in das Plasmid pADH2Hir_ins_glyA21 klonierten Insulinanaloga - Sequenz. In einer PCR (Standardbedingungen) wird ein DNA Fragment generiert, das nach Verdau mit den Restriktionsenzymen Kpnl und NcoI in den entsprechend geöffneten Expressionsvektor insertiert wird und das gewünschte Fusionsprotein enthält. Das in kompetente Zellen des Hefestammes Y79 transformiert. Transformanten werden anschließend wie in Beispiel 7 beschrieben exprimiert. Das Arg(B31), Gly(A21) - Miniproinsulin wird nach bekannten Methoden (EP-A 0 229 998) isoliert und gemäß Beispiel 2 in Arg(B31), Lys(B32), Gly(A21) Humaninsulin umgewandelt.

### Beispiel 9: Gensequenz zu Sekretion eines Hirudin - Arg(B31), Gly(A21) Humaninsulin -Fusionsproteins durch Pichia pastoris

Die Klonierung des Expressionsvektors erfolgt in Analogie zu Beispiel 4 der Patentanmeldung EP-A 1 364 032. Anstelle der Sequenz Primer pichia_H_Irev2 wird dabei der Primer ins_gly_rev2 der später die Möglichkeit der Expression von Gly(A21) Humaninsulin mit dem PCR-Produkt ermöglicht, eingesetzt:
5'- TTTTTGGCGCCGAATTCACTACTATTAGCCACAGTAGTTTTCCAGCTGG - 3' (SEQ ID NO.:6)

Das entstandene Plasmid erhält die Bezeichnung pPich_Hir_ins-GlyA21. Die Reinigung von Arg(B31), Gly(A21) - Miniproinsulin als Ausgangsmaterial zur Erzeugung eines Analogons mit dibasischem Kettende wird wie beschrieben durchgeführt.

Beispiel 10: Gensequenz zur direkten Sekretion der Arg(B31), Gly(A21) - Vorstufe durch Pichia pastoris

In Analogie zu Beispiel 7 erfolgt die Konstruktion des entsprechenden Expressionsvektors. Man benötigt die DNA des Plasmides pPich_Hir_ins-GlyA21 und zwei Primer pich_insgly_dirf und pich_insgly_dirrev
pich_insgly_dirf
   5'-TTTTTTCTCGAGAAAAGATTTGTTAACCAACACTTGTGTG-3' (SEQ ID NO.: 7)
pich_insgly_dirrev
   5'-TTTTTT GGCGCCGAATTCACTACTATTAGCCAC-3' (SEQ ID NO.: 8)

### Beispiel 11: Herstellung von Arg(B31), Gly(A21) - Insulin aus einem durch Hefe - Sekretion gewonnenen Fusionsproteins, das Proinsulin korrekt gefaltet enthält und dessen Fusionsteil eine His₆ - Aminosäurefolge enthält

DNA des Plasmides pADH2Hir_ins_glyA21 dient als Template. Zwei Primer werden synthetisiert:
alpha_LT_H6_hirf und alpha_LT_H6_hirrev
alpha_LT_H6_hirt1 :
   5'- GCACCATCATCACCATCACTATACTGACTGCACTGAATC -3' (SEQ ID NO.: 9)
   Der Primer enthält die Codone für 6 Histidine in Reihe und die Aminosäuren 3- 8 sowie 9 (partial) der Refludan^{®} - Sequenz.
alpha_LT_H6_hirf2 :
   5'- GAAGGGGTACCTTTGGATAAAAGACTTACGCACCATCATCACCATCAC -3' (SEQ ID NO.: 10)

Der Primer enthält die Codone für 6 Histidine in Reihe, die Codone für die Aminosäuren 1 und 2 der Lepirudin - Sequenz und alpha - Faktorsequenzen, die die Lys - Arg Prozessierungsstelle umfassen und die Erkennungsstelle für das Restriktionsenzym Kpn 1 überdecken. DNA des Plasmides pADH2Hir_ins_glyA21 dient als Template in einer Standard PCR mit den Primern alpha_LT_H6_hirf1 und ins_gly_a21_rev aus Beispiel 7 der vorliegenden Anmeldung. Das Produkt der Reaktion wird isoliert und ein Aliquot als Template für eine zweite PCR mit den Primern alpha_LT_H6_hirf2 und ins_gly_a21_rev eingesetzt. Das Reaktionsprodukt wird wie beschrieben mit KPN1 und Nco1 prozessiert und dann kloniert. Es entsteht da Plasmid pADH2_LT_H6_Hir_ins_glyA21 : Nach Transformation von Y79 mit DNA des Plasmides wird das Fusionsprotein exprimiert. Durch Zentrifugation werden die Zellen von dem Überstand abgetrennt und der Überstand über Membranfilter z.B- der Firma Sartorius konzentriert und anschließend über Ni²⁺ - Affinitätschromatographie, wobei man der Vorschrift zum ProBond TM Reinigungssystem der Firma Invitrogen folgt. Nach Entfernung des Elutionspuffer über Dialsyse und/ oder Filtration bzw. Gelfiltration als Alternative kann das Fusionprotein in bekannter Weise zu Arg (B31), Gly (A21) Humaninsulin prozessiert und anschließend zu Insulin Glargin umgewandelt werden.

### Beispiel 12 : Herstellung von Arg (B31), Gly (A21) Humaninsulin aus einem durch Hefe - Sekretion gewonnenen Fusionsproteins, das Proinsulin korrekt gefaltet enthält und dessen Fusionsteil mit dem Enzym Enterokinase abgespalten wird

DNA des Plasmides pADH2Hir_ins_glyA21 dient als Ausgangsmaterial. Die Primer ins_gly_a21_rev aus Beispiel 7 der vorliegenden Anmeldung und hirf1aus Beispiel 1 der Anmeldung WO 02/070722 A1 werden verwendet. Dazu werden zwei neue Primer hergestellt:
Hir_entero_insf
   5'- CTTCAG GACGATGACGATAAATTTGTTAACCAACACTTGTGTGG-3' (SEQ ID NO.: 11)

Der Primer überdeckt die Aminosäuren B1- B7 und B8 (partial) der Miniproinsulinsequenz und enthält die Codone für die Aminosäuresequenz Asp- Asp-Asp-Asp-Lys, die Erkentennungsstelle für Enterokinase darstellen.
Hir_entero_insrev
   5'- TTTATCGTCATCGTCCTGAAGGCTGAAGGTATTCCTCAGGG-3' (SEQ ID NO.: 12)

Der reverse Primer überdeckt die Aminosäuren 60-65 der Lepirudin - Sequenz und enthält die Codone für die Aminosäuresequenz Asp- Asp-Asp-Asp-Lys (SEQ ID NO.:13), die Erkentennungsstelle für Enterokinase darstellen. Es werden zunächst zwei PCR mit den Primerpaaren hirf1/ Hir_entero_insrev und Hir_entero_insf/ins_gly_a21_rev durchgeführt. Die Raktionsprodukte werden isoliert. Aliquots des Materials werdn gemischt und die Mischung in einer dritten PCR als Template für das Primerpaar hirf1/ ins_gly_a21_rev eingesetzt. Das Reaktionsprodukt wird wie beschrieben kloniert. Es entseht der Vektor pADH2Hir_ins_glyA21. Das Fusionsprotein wird wie beschrieben hergestellt.

Die Spaltung des Fusionsproteins erfolgt über Enterokinase . Das Enzym ist kommerziell erhältlich.

Die Spaltungsreaktion wurde in Enterokinase-Puffer (20 mM Tris/HCl, 50 mM NaCl, 2 mM CaCl₂ pH 7,4) unter Einsatz einer der jeweiligen Herstellerangabe entsprechenden Menge Enzym durchgeführt.. Die Spaltung erfolgt in der Regel nach dem Abtrennen der Wirtszellen und dem folgenden Aufarbeitungsschritt. Sie kann aber auch direkt im Überstand nach Fermentation erfolgen, nachdem die optimalen Reaktionsbedingungen eingestellt wurden.

### Beispiel 13 : Herstellung von Arg (B31), Gly (A21) Humaninsulin aus einem durch Hefe - Sekretion gewonnenen Fusionsproteins, das Proinsulin korrekt gefaltet enthält und dessen Fusionsteil mit dem Enzym Enterokinase abgespalten wird und eine poly - Histidin -Sequenz enthält

Man verwendet DNA des Plasmides pADH2_LT_H6_Hir_ins_glyA21 und die Primer Hir_entero_insrev, Hir_entero_insf und ins_gly_a21_rev und ersetzt Primer hirf1 durch den Primer alpha_It-enterof mit folgender Sequenz:
5'- GAAGGGGTACCTTTGGATAAAAG - 3' (SEQ ID NO.: 13)

In Analogie zu Beispiel 12 konstruiert man dann einen Vektor pADH2_LT_H6_Hir_etero_ins_glyA21 der für eine Fusionsprotein kodiert, dessen Hirudinfusionsteil um sechs Histidine beginnend mit Position 3 N-terminal und C-terminal ab Position 72 um die Sequenz DDDDK (SEQ ID NO.: 14) erweitert wurde. Die Herstellung von Arg (B31), Gly (A21) Humaninsulin erfolgt dann durch Kombination der in den Beispielen 11 und 12 beschriebenen Methode.

### Beispiel 14: Gensequenz zu Sekretion eines Hirudin des Phe (B1),Arg(B31), Gly(A21) Insulin - Fusionsproteins durch Bäckerhefe

Die Transformation und Expression erfolgt in Analogie zu Beispiel 7.

Zwei Primersequenzen werden synthetisiert:
Desphef1:
   5'-CTTCAGGGAAATTCGGCACGAGTTAACCAACACTTGTGTGGTTC-3' (SEQ ID NO.: 15)
und Desphe_rev1:
   5'-GAACCACACA AGTGTTGGTT AACTCGTGCC GAATTTCCCT GAAG-3' (SEQ ID NO.: 16)

DNA des Plasmides pADH2Hir_ins_glyA21 aus Beispiel 7 dient als Template. Zwei Polymerasekettenreaktionen werden unabhängig voneinander durchgeführt. In Reaktion 1 werden die Primer Desphe_rev1 und der Primer SEQ ID NO:4 aus Beispiel 1 der Anmeldung EP-A 1 364 032 und in Reaktion 2 der Primer ins_gly_a21_rev aus Beispiel 7 der vorliegenden Anmeldung und der Primer Desphef1 eingesetzt. Die Reaktionsprodukte beider Reaktionen werden isoliert und Aliquots der Ausbeute in einer dritten Reaktion vereinigt und als Template für das Primerpaar bestehend aus den Primern SEQ ID NO:4 aus Beispiel 1 der Anmeldung EP-A 1 364 032 und ins_gly_a21_rev eingesetzt. Das Reaktionsprodukt der dritten Reaktion wird wie in Beispiel 7 beschrieben kloniert, transformiert und exprimiert. Das entstandene Fusionsprotein dient als Ausgangsmaterial zur Herstellung von entsprechenden Insulinanaloga mit di- basischen Kettenenden.

### Beispiel 15: Gensequenz zu Sekretion eines Hirudin Ala (B31),Arg(B32), Gly(A21) Insulin - Fusionsproteins durch Bäckerhefe

Zwei Primersequenzen werden synthetisiert:
Ala_b31f1:
   5'-CTTCTACACTCCAAAGACGgctCGTGGTATCGTTGAACAATGTTG-3' (SEQ ID NO.: 17)
und Ala_b31 rev1:
   5'-CAACATTGTT CAACGATACC ACGagcCGTC TTTGGAGTGT AGAAG-3' (SEQ ID NO.: 18)

DNA des Plasmides pADH2Hir_ins_glyA21 aus Beispiel 7 dient als Template. Zwei Polymerasekettenreaktionen werden unabhängig voneinander durchgeführt. In Reaktion 1 werden die Primer Ala_b31rev1 und der Primer SEQ ID NO:4 aus Beispiel 1 der Anmeldung EP-A 1 364 032 und in Reaktion 2 der Primer ins_gly_a21_rev aus Beispiel 7 der vorliegenden Anmeldung und der Primer Ala_b31f1 eingesetzt. Die Reaktionsprodukte beider Reaktionen werden isoliert. Aliquots der Ausbeute werden in einer dritten Reaktion vereinigt und als Template für das Primerpaar bestehend aus den Primern SEQ ID NO:4 aus Beispiel 1 der Anmeldung EP-A 1 364 032 und ins_gly_a21_rev eingesetzt. Das Reaktionsprodukt der dritten Reaktions wird wie in Beispiel 7 beschrieben kloniert, transformiert und exprimiert. Das entstandene Fusionsprotein dient als Ausgangsmaterial zur Herstellung von entsprechenden Insulinanaloga mit di- basischen Kettenenden.

### Beispiel 16: Gensequenz zur direkten Sekretion einer Lys(B31), - Vorstufe durch Bäckerhefe.

Zwei Primer werden synthetisiert:
Lys_b31f
5'-CTTCTACACTCCAAAGACGAAAGGTATCGTTGAACAATGTTG-3' (SEQ ID NO.: 19)
und Lys_b31 rev
5'-CAACATTGTT CAACGATACC TTTCGTCTTT GGAGTGTAGA AG -3 (SEQ ID NO.: 20)

DNA des Plasmides pADH2Hir_ins aus Beispiel 1 der Anmeldung WO 02/070722A1 dient als Template für zwei Polymerasekettenrektionen. In Reaktion 1 werden die Primer Lys_b31f1 und insnco1 rev (Seq ID NO:6 aus WO 02/070722A1) und in Reaktion 2 die Primer Lys_b31 rev und alpha_insf1 aus Beispiel 7 der vorliegenden Anmeldung eingesetzt. Die Standardreaktionen werden durchgeführt und die entstandenen PCR - Fragmente isoliert. Aliquots der beiden Ausbeuten werden vereinigt und dienen als Template für eine dritte Reaktion mit den Primern insnco1 rev und Seq ID NO:6 aus WO 02/070722A1. Das entstandene PCR - Fragment wird wie in Beispiel 8 beschrieben kloniert und exprimiert. Es entsteht Lys(B31) -Miniproinsulin, das mit Lysylendopeptidase in B(1-29) - A(1-21) Split Insulin umgewandelt wird und als Intermediate zur Herstellung von B30 - Argininamid insulin oder B30 Lysysinamid - Insulin, das anschließend zu dem jeweiligen dibasischen Analogon umgewandelt werden kann.

### Beispiel 17 :Spaltung mit Lysylendopeptidase:

Die Insulinvorstufe wird wie in DE3844211 beschrieben mit Lysylendopeptidase (LEP) umgesetzt (Beispiel 1). Hierzu werden 10 mg Lys(B31)-Miniproinsulin in Tris-Puffer (pH 8.0) gelöst und LEP aus Lysobacter enzymogenes zugegeben (0.01 ml einer 1 mg/ml konz. Lösung in Wasser, Merckbiosciences). Es wird bei Raumtemperatur 2 h inkubiert und über RP-HPLC aufgereinigt (Nucleosil 120-5 Säule). Es entsteht B(1-29) - A(1-21) Split Insulin.

### Beispiel 18: Herstellung von Arg(B30) -Insulin aus einer B(1-29)-A(1-21) Splitinsulinvorstufe über Kupplung mit Argininamid

100 mg B(1-29) - A(1-21) Split Insulin werden in 0.95 ml Argininamidlösung (446 g/L) gelöst , 0.13 mL M Na-Acetatpuffer (pH 5.8) und 2 ml DMF zugegeben. Die Reaktionsmischung wird auf 12° C gekühlt und durch Zugabe von 0.094 ml Trypsin (0.075 mg, Roche Diagnostics) gestartet.

Nach 8 h wird die Reaktion durch Zugabe von TFA bis pH 2.5 gestoppt und per HPLC analysiert. Es bildet sich> 60 % Arg(B30) -Insulinamid. Nach Zusatz von Trypsininhibitorlösung erfolgt die Reinigung des amidierten Analogs in Analogie zu US 5,656,722. Das amidierte Insulinanalog kann anschließend in Gegenwart von Säure mehrere Stunden zu Arg(B30)insulin hydrolysiert werden oder das Amid kann direkt als Arzneimittel eingesetzt werden.

### Beispiel 19: Herstellung von Lys(B30) -Insulin aus einer B(1-29)-A(1-21) Splitinsulinvorstufe über Kupplung mit Lysinamid

100 mg B(1-29) - A(1-21) Split Insulin werden in 0.93 ml Lysinamidlösung (400 g/L) gelöst , 0.13 mL M Na-Acetatpuffer (pH 5.8) und 2 ml DMF zugegeben. Die Reaktionsmischung wird auf 12° C gekühlt und durch Zugabe von 0.094 ml Trypsin (0.075 mg, Roche Diagnostics) gestartet. Nach 8 h wird die Reaktion durch Zugabe von TFA bis pH 2.5 gestoppt und per HPLC analysiert. Es bildet sich Lys(B30)-Insulinamid, das nach Zusatz von Trypsininhibitorlösung in Analogie zu US 5,656,722 gereinigt wird. Das amidierte Insulinanalog kann anschließend in Gegenwart von Säure mehrere Stunden zu Lys(B30)-Insulin hydrolysiert werden oder direkt als Arzneimittel eingesetzt werden.

## Patentansprüche

1. Verfahren zur Herstellung eines Insulinanalogons oder eines Derivates davon, wobei das Insulinanalogon charakterisiert ist durch die allgemeine Formel I 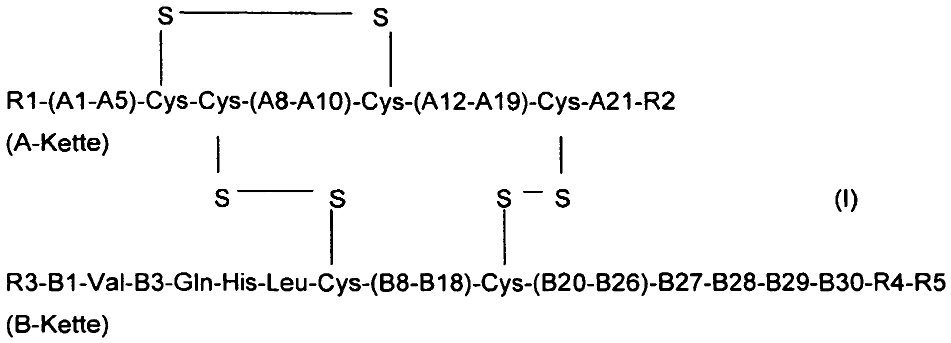 worin bedeuten
| | |
|---|---|
| (A1 -A5) | die Aminosäurereste in den Positionen A1 bis A5 der A-Kette von Humaninsulin oder tierischem Insulin, |
| | |
| (A12-A19) | die Aminosäurereste in den Positionen A12 bis A19 der A-Kette von Humaninsulin oder tierischem Insulin, |
| | |
| A21 | ein natürlich auftretender Aminosäurerest, |
| | |
| (B8-B18) | die Aminosäurereste in den Positionen B8 bis B18 der B-Kette von Humaninsulin oder tierischem Insulin, |
| (B20-B26) | die Aminosäurereste in den Positionen B20 bis B26 der B-Kette von Humaninsulin oder tierischem Insulin, |
| | |
| (A8-A10) | die Aminosäurereste in den Positionen A8 bis A10 der A-Kette von Humaninsulin oder tierischem Insulin, |
| B30 | eine chemische Bindung oder ein natürlich auftretender Aminosäurerest, |
| | |
| B1 | eine chemische Bindung oder ein natürlich auftretender Aminosäurerest, |
| | |
| B3 | ein natürlich auftretender Aminosäurerest, |
| | |
| B27, B28 und B29 | ein natürlich auftretender Aminosäurerest, |
| | |
| R1 | eine Aminogruppe oder ein bis drei natürlich auftretende Aminosäurereste, |
| | |
| R2 | eine Carboxygruppe oder ein bis drei natürlich auftretende Aminosäurereste, |
| | |
| R3 | eine Aminogruppe oder ein bis drei natürlich auftretende Aminosäurereste, |
| | |
| R4 | eine basische Aminosäure darstellt, |
| | |
| R5 | ein basischer Aminosäurerest, dessen C-Terminus entweder frei oder amidiert vorliegt, |
wobei derjenige Aminosäurerest, an dessen C-Terminus der N-Terminus von R5 anschließt, ausgewählt ist aus einer Gruppe enthaltend Arg und Lys;
bei dem
an ein Ausgangs-Insulinanalogon oder ein Derivat davon, wobei das Ausgangs-Insulinanalogon charakterisiert ist durch die allgemeine Formel II 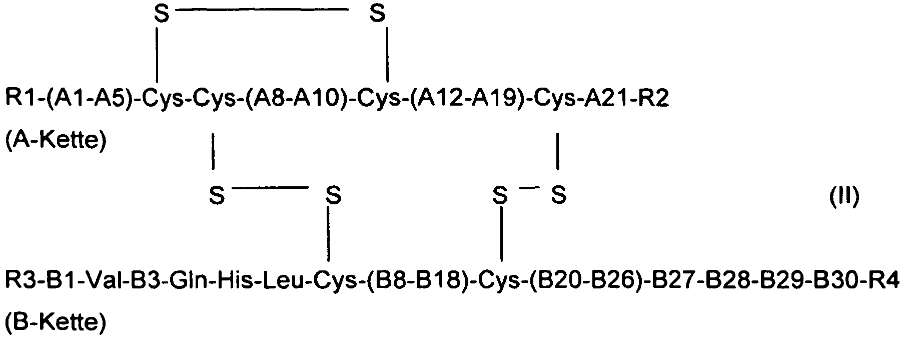 wobei R1, (A1-A5), (A8-A10), (A12-A19), A21, R2, R3, B1, B3, (B8-B18), (B20-B26), B27, B28, B29, B30 und R4 definiert sind wie in der Formel I und der C-terminale Aminosäurerest der B-Kette ausgewählt ist aus einer Gruppe enthaltend Arg und Lys,
an eine der genannten C-terminalen Aminosäuren in Gegenwart eines Enzyms mit der biologischen Aktivität von Trypsin, eine amidierte oder C-terminal mit einer Schutzgruppe geschützte, natürlich vorkommende, basische Aminosäure ausgewählt aus der Gruppe Arg und Lys addiert,
und das erhaltene modifizierte Insulinanalogon aufreinigt und optional die Amidgruppe oder C-terminale Schutzgruppe der addierten Aminosäure abspaltet.

2. Verfahren gemäß Anspruch 1, wobei das modifizierte Insulinanalogon Gly(A21), Arg(B31), Arg(B32) Humaninsulin ist, dessen C-terminales Ende der B-Kette amidiert ist.

3. Verfahren gemäß Anspruch 2, bei dem das Ausgangs-Insulinanalogon Gly(A21), Arg(B31) Humaninsulin ist.

4. Verfahren gemäß einem der Ansprüche 1 bis 3, wobei das Ausgangs-Insulinanalogon hergestellt wird durch rekombinante Expression eines Vorläuferproteins enthaltend die A- und die B-Kette des Ausgangs-Insulinanalogons.

5. Verfahren gemäß Anspruch 4, wobei ein Gen, welches Teil eines Replicons ist, exprimiert wird.

6. Verfahren gemäß einem der Ansprüche 4 oder 5, wobei als Wirtszelle ein Bakterium oder eine Hefe verwendet wird.

7. Verfahren gemäß einem der Ansprüche 4 bis 6, wobei das Vorläuferprotein nach der Expression sekretiert wird.

8. Verfahren gemäß Anspruch 7, wobei das Vorläuferprotein aus dem zellulären Überstand von Bakterien oder Hefen isoliert wird.

9. Verfahren zur Herstellung von modifizierten Insulinanaloga gemäß Anspruch 6 wobei das Vorläuferprotein aus dem Periplasma eines Bakteriums isoliert wird.

10. Verfahren gemäß einem der Ansprüche 4 bis 7, wobei das nach einem der genannten Ansprüche erhaltene Vorläuferprotein einem Faltungsprozeß und enzymatischer Spaltung unterworfen wird.

11. Verfahren gemäß einem der Ansprüche 1 bis 3, wobei das Ausgangs-Insulinanalogon hergestellt wird durch rekombinante Direktexpression.

12. Verfahren gemäß einem oder mehreren der Ansprüche 1 bis 11, wobei das Enzyms mit der biologischen Aktivität von Trypsin ausgewählt ist aus einer Gruppe enthaltend menschliches Trypsin, Schweinetrypsin, Rindertrypsin und eine Variante von menschlichem Trypsin, Schweinetrypsin und Rindertrypsin.

13. Verfahren gemäß einem oder mehreren der Ansprüche 1 bis 11, wobei das Enzym Lysylendopeptidaseaktivität aufweist

14. Verfahren gemäß einem oder mehreren der Ansprüche 1 bis 12, wobei das C-terminale Ende der B-Kette des modifizierten Insulinanalogons nachfolgend in einer Hydrolysereaktion entschützt wird.

15. Verfahren gemäß einem oder mehreren der Ansprüche 1 bis 14, bei dem das erhaltene Insulinanalogon Gly(A21), Arg(B31), Arg(B32) Humaninsulin ist.

## Claims

1. A method for preparing an insulin analog or a derivative thereof, where the insulin analog is **characterized by** the general formula I 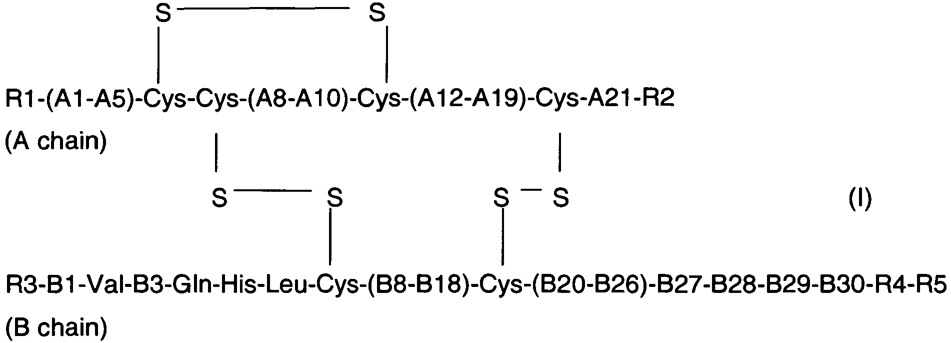 in which the meanings are
| | |
|---|---|
| (A1-A5) | the amino acid residues in positions A1 to A5 of the A chain of human insulin or animal insulin, |
| | |
| (A12-A19) | the amino acid residues in positions A12 to A19 of the A chain of human insulin or animal insulin, |
| | |
| A21 | a naturally occurring amino acid residue, |
| (B8-B18) | the amino acid residues in positions B8 to B18 of the B chain of human insulin or animal insulin, |
| | |
| (B20-B26) | the amino acid residues in positions B20 to B26 of the B chain of human insulin or animal insulin, |
| | |
| (A8-A10) | the amino acid residues in positions A8 to A10 of the A chain of human insulin or animal insulin, |
| | |
| B30 | a chemical bond or a naturally occurring amino acid residue, |
| | |
| B1 | a chemical bond or a naturally occurring amino acid residue, |
| | |
| B3 | a naturally occurring amino acid residue, |
| | |
| B27, B28 and B29 | a naturally occurring amino acid residue, |
| | |
| R1 | an amino group or one to three naturally occurring amino acid residues, |
| | |
| R2 | a carboxy group or one to three naturally occurring amino acid residues, |
| | |
| R3 | an amino group or one to three naturally occurring amino acid residues, |
| | |
| R4 | a basic amino acid, |
| | |
| R5 | basic amino acid residue whose C terminus is either free or amidated, |
where the amino acid residue whose C terminus is connected to the N terminus of R5 is selected from a group comprising Arg and Lys;
in which
a naturally occurring, basic amino acid which is amidated or protected C-terminally with a protective group and is selected from the group Arg and Lys is added
onto an initial insulin analog or a derivative thereof, where the initial insulin analog is **characterized by** the general formula II 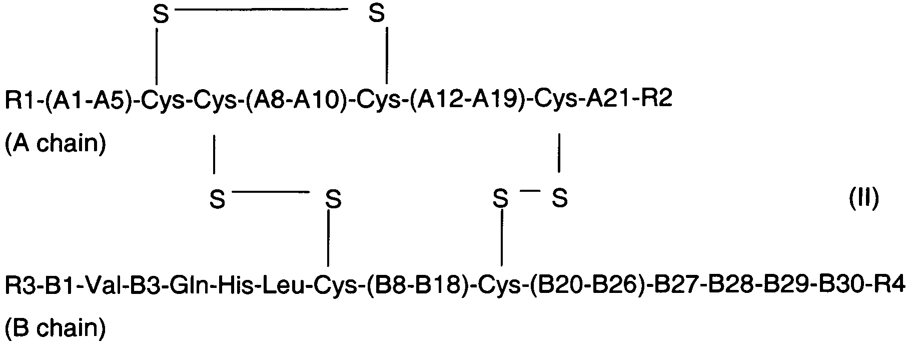 where R1, (A1-A5), (A8-A10), (A12-A19), A21, R2, R3, B1, B3, (B8-B18), (B20-B26), B27, B28, B29, B30 and R4 are defined as in the formula I, and the C-terminal amino acid residue of the B chain is selected from a group comprising Arg and Lys,
onto one of said C-terminal amino acids in the presence of an enzyme having the biological activity of trypsin,
and the resulting modified insulin analog is purified and optionally the amide group or C-terminal protective group of the added amino acid is eliminated.

2. The method as claimed in claim 1, where the modified insulin analog is Gly(A21), Arg(B31), Arg(B32) human insulin whose C-terminal end of the B chain is amidated.

3. The method as claimed in claim 2, in which the initial insulin analog is Gly(A21), Arg(B31) human insulin.

4. The method as claimed in any of claims 1 to 3, where the initial insulin analog is prepared by recombinant expression of a precursor protein comprising the A chain and the B chain of the initial insulin analog.

5. The method as claimed in claim 4, where a gene which is part of a replicon is expressed.

6. The method as claimed in either of claims 4 or 5, where a bacterium or a yeast is used as host cell.

7. The method as claimed in any of claims 4 to 6, where the precursor protein is secreted after expression.

8. The method as claimed in claim 7, where the precursor protein is isolated from the cellular supernatant of bacteria or yeasts.

9. The method for preparing modified insulin analogs as claimed in claim 6, where the precursor protein is isolated from the periplasm of a bacterium.

10. The method as claimed in any of claims 4 to 7, where the precursor protein obtained as claimed in any of said claims is subjected to a folding process and enzymatic cleavage.

11. The method as claimed in any of claims 1 to 3, where the initial insulin analog is prepared by recombinant direct expression.

12. The method as claimed in one or more of claims 1 to 11, where the enzyme having the biological activity of trypsin is selected from a group comprising human trypsin, porcine trypsin, bovine trypsin and a variant of human trypsin, porcine trypsin and bovine trypsin.

13. The method as claimed in one or more of claims 1 to 11, where the enzyme has lysyl endopeptidase activity.

14. The method as claimed in one or more of claims 1 to 12, where the C-terminal end of the B chain of the modified insulin analog is subsequently deprotected in a hydrolysis reaction.

15. The method as claimed in one or more of claims 1 to 14, in which the resulting insulin analog is Gly(A21), Arg(B31), Arg(B32) human insulin.

## Revendications

1. Procédé de préparation d'un analogue de l'insuline ou d'un dérivé de ce dernier, l'analogue de l'insuline étant **caractérisé par** la formule générale I 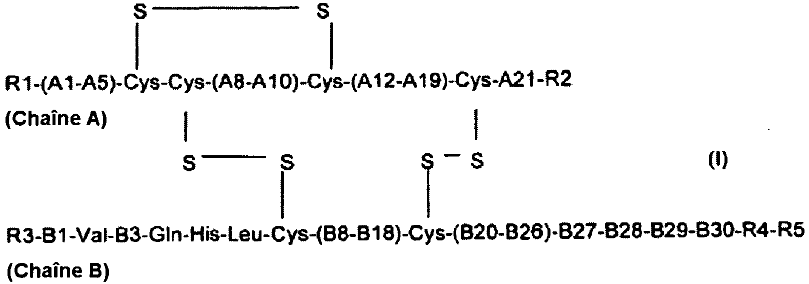 dans laquelle
(A1-A5) représentent les résidus d'acides aminés en positions A1 à A5 de la Chaîne A de l'insuline humaine ou de l'insuline animale,
(A12-A19) représentent les résidus d'acides aminés en positions A12 à A19 de la Chaîne A de l'insuline humaine ou de l'insuline animale,
A21 est un résidu d'acide aminé naturel,
(B8-B18) représentent les résidus d'acides aminés en positions B8 à B18 de la Chaîne B de l'insuline humaine ou de l'insuline animale,
(B20-B26) représentent les résidus d'acides aminés en positions B20 à B26 de la Chaîne B de l'insuline humaine ou de l'insuline animale,
(A8-A10) représentent les résidus d'acides aminés en positions A8 à A10 de la Chaîne A de l'insuline humaine ou de l'insuline animale,
B30 représente une liaison chimique ou un résidu d'acide aminé naturel,
B1 représente une liaison chimique ou un résidu d'acide aminé naturel,
B3 représente un résidu d'acide aminé naturel,
B27, B28 et B29 représentent chacun un résidu d'acide aminé naturel,
R1 représente un groupe amino ou un à trois résidus d'acides aminés naturels,
R2 représente un groupe carboxy ou un à trois résidus d'acides aminés naturels,
R3 représente un groupe amino ou un à trois résidus d'acides aminés naturels,
R4 représente un acide aminé basique,
R5 représente un résidu d'acide aminé basique dont le site C-terminal est présent sous forme libre ou amidée,
dans lequel le résidu d'acide aminé dont le site C-terminal est suivi du site N-terminal de R5 est choisi dans un groupe contenant Arg et Lys ;
dans lequel :
à un analogue de l'insuline de départ ou à un dérivé de ce dernier, l'analogue de l'insuline de départ étant **caractérisé par** la formule générale II 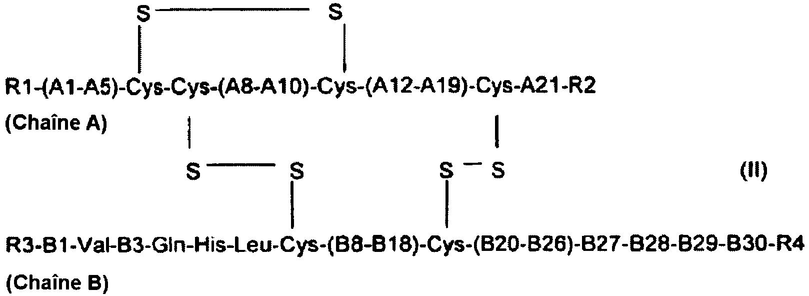 R1, (A1-A5), (A8-A10), (A12-A19), A21, R2, R3, B1, B3, (B8-B18), (B20-B26), B27, B28, B29, B30 et R4 étant définis comme dans la Formule I, et le résidu d'acide aminé C-terminal de la Chaîne B étant choisi dans un groupe contenant Arg et Lys ;
on ajoute à l'un des acides aminés C-terminaux mentionnés, en présence d'une enzyme ayant l'activité biologique de la trypsine, un acide aminé basique naturel choisi dans le groupe Arg et Lys, amidé ou protégé en position C-terminale par un groupe protecteur,
et on purifie l'analogue de l'insuline modifié obtenu, et en option on dissocie de l'acide aminé ajouté le groupe amide ou le groupe protecteur C-terminal.

2. Procédé selon la revendication 1, dans lequel l'analogue de l'insuline modifié est la Gly(A21), Arg(B31), Arg(B32)-insuline humaine, dont l'extrémité C-terminale de la Chaîne B est amidée.

3. Procédé selon la revendication 2, dans lequel l'analogue de l'insuline de départ est la Gly(A21), Arg(B31)-insuline humaine.

4. Procédé selon l'une des revendications 1 à 3, dans lequel l'analogue de l'insuline de départ est préparé par expression recombinante d'une protéine précurseur contenant la Chaîne A et la Chaîne B de l'analogue de l'insuline de départ.

5. Procédé selon la revendication 4, dans lequel on exprime un gène qui est une partie d'un réplicon.

6. Procédé selon l'une des revendications 4 ou 5, dans lequel on utilise en tant que cellule hôte une bactérie ou une levure.

7. Procédé selon l'une des revendications 4 à 6, dans lequel la protéine précurseur est sécrétée après l'expression.

8. Procédé selon la revendication 7, dans lequel la protéine précurseur est isolée du surnageant cellulaire de bactéries ou de levures.

9. Procédé de préparation d'analogues de l'insuline modifiés selon la revendication 6, dans lequel la protéine précurseur est isolée du périplasme d'une bactérie.

10. Procédé selon l'une des revendications 4 à 7, dans lequel la protéine précurseur obtenue d'après l'une des revendications mentionnées est soumise à un processus de repliement et à un clivage enzymatique.

11. Procédé selon l'une des revendications 1 à 3, dans lequel l'analogue de l'insuline de départ est préparée par expression directe recombinante.

12. Procédé selon l'une ou plusieurs des revendications 1 à 11, dans lequel l'enzyme ayant l'activité biologique de la trypsine est choisie dans un groupe contenant la trypsine humaine, la trypsine de porc, la trypsine de bovin et un variant de la trypsine humaine, de la trypsine de porc et de la trypsine de bovin.

13. Procédé selon l'une ou plusieurs des revendications 1 à 11, dans lequel l'enzyme présente une activité de lysylendopeptidase.

14. Procédé selon l'une ou plusieurs des revendications 1 à 12, dans lequel l'extrémité C-terminale de la Chaîne B de l'analogue de l'insuline modifié est ensuite déprotégée dans une réaction d'hydrolyse.

15. Procédé selon l'une ou plusieurs des revendications 1 à 14, dans lequel l'analogue de l'insuline ainsi obtenu est la Gly(A21), Arg(B31), Arg(B32)-insuline humaine.
